# EUROPÄISCHE PATENTSCHRIFT

(11) **EP 4 137 098 B1**
(45) Veröffentlichungstag und Bekanntmachung des Hinweises auf die Patenterteilung: **25.10.2023**
(21) Anmeldenummer: 21000233.3
(22) Anmeldetag: 16.08.2021
(51) Int. Cl.: A61F 2/30

(54) **SCHEIBENFÖRMIGES AUGMENT FÜR EINEN RÖHRENKNOCHEN**
DISC-SHAPED AUGMENT FOR A TUBULAR BONE
INSERT EN FORME DE DISQUE POUR UN OS TUBULAIRE

(43) Veröffentlichungstag der Anmeldung: 22.02.2023
(73) Patentinhaber: Waldemar Link GmbH & Co. KG, 22339 Hamburg (DE)
(72) Erfinder: JENSEN, Harm-Iven, 24214 Noer (DE); LINK, Helmut D., 22397 Hamburg (DE)
(74) Vertreter: Glawe, Delfs, Moll

(56) Entgegenhaltungen:
- EP-B1- 1 360 950
- DE-A1-102006 047 663
- US-A1- 2012 209 391

## Beschreibung

Die Erfindung betrifft ein scheibenförmiges Augment zum Ausfüllen von Knochendefekten, insbesondere am Ende von langen Röhrenknochen wie Tibia. Das Augment ist versehen mit einer proximalen Seite, einer distalen Seite, einem Außenmantel an Lateralseiten sowie einer Innenwandung für eine von der proximalen zur distalen Seite laufende Durchgangsöffnung für einen Verankerungskiel einer an der proximalen Seite angeordneten Endoprothese.

Bei der Implantation von Endoprothesen, insbesondere Gelenkendoprothesen, tritt mitunter ein Problem auf, dass der die Endoprothese aufnehmende Knochen geschädigt ist, und zwar speziell im Bereich des Endes des Knochens (Knochenkopf). Grund hierfür sind insbesondere Defekte an der (spongiösen und/oder kortikalen) Knochensubstanz oder -oberfläche, bspw. aufgrund von Krankheit oder Verletzung, zunehmend aber auch aufgrund einer Explantation einer älteren Prothese. Um dennoch eine ausreichende Basis im Knochen zum Verankern der Endoprothese zu schaffen, werden typischerweise Augmente eingesetzt, die fehlende Knochensubstanz auffüllen. Es hat sich bewährt, sie versenkt im Knochenende anzuordnen, so dass sie von dem kortikalen Rand des Knochenendes köcherartig umgeben sind.

Scheibenartige Augmente zur Anwendung mit der Tibiakomponente einer Kniegelenkendoprothese sind bekannt beispielsweise aus der EP 1 360 950 B1. Vorgesehen sind unterhalb der Tibiaplatte zwei halbseitige Augmente, die links und rechts eines Verankerungskiels angeordnet sind. Die Augmente liegen bündig an der Unterseite der Tibiaplatte an und sind im montierten Zustand dort mittels einer Befestigungsschraube gesichert. Es hat sich gezeigt, dass auf diese Weise die Augmente zwar definiert in Bezug auf die Endoprothese gehaltert sind, jedoch nicht in Bezug auf den Knochenkopf, den sie eigentlich stützen bzw. dessen Knochendefekt sie an sich ausfüllen sollen. Eine weitere Schwierigkeit kann nach längerem Einsatz dadurch entstehen, dass im Laufe der Zeit das Augment im Knochen einwächst und danach die Endoprothese mit dem Augment, beispielsweise im Rahmen einer Revisionsoperation, nur schwer oder kaum zu entfernen ist. Es besteht dabei die Gefahr einer weiteren Schädigung des Knochens am ohnehin empfindlichen Knochenkopf.

Der Erfindung liegt die Aufgabe zugrunde, ein verbessertes Augment zu schaffen, mit dem die vorgenannten Nachteile vermieden oder verringert werden können.

Die erfindungsgemäße Lösung liegt in den Merkmalen des unabhängigen Anspruchs. Vorteilhafte Weiterbildungen sind Gegenstand der abhängigen Ansprüche.

Bei einem scheibenförmigen Augment zum Ausfüllen von Knochendefekten, insbesondere am Ende von langen Röhrenknochen wie Tibia, mit einer proximalen Seite, einer distalen Seite, einem Außenmantel an Lateralseiten sowie einer Innenwandung für eine von der proximalen zur distalen Seite laufende Durchgangsöffnung für einen Verankerungskiel einer an der proximalen Seite angeordneten Endoprothese, wobei das Augment eine generell C-förmige Gestalt mit zwei die Durchgangsöffnung flankierenden Schenkeln aufweist, ist erfindungsgemäß vorgesehen, dass ein Verbindungsstück zwischen den Schenkeln gelenkartig ausgeführt ist und mit den Schenkeln elastisch zusammenwirkt, so dass bei Kompression der Schenkel eine nach außen gerichtete Rückstellkraft erzeugt ist, und der Außenmantel als eine poröse, Knocheneinwuchs fördernde Struktur ausgeführt ist sowie die proximale Seite mit einer Deckplatte versehen ist, die eine geriffelte Struktur als Verzahnung für eine Zementverbindung aufweist.

Nachfolgend seien zuerst einige verwendete Begriffe erläutert:
Unter der Kompression der Schenkel wird eine Bewegung der Schenkel aufeinander zu verstanden, wobei ein zwischen ihnen befindlicher Freiraum verengt wird.

Unter einer geriffelten Struktur wird vorzugsweise eine grob geriffelte Struktur verstanden, mit einem Abstand zwischen einzelnen Profilen der Riffelung von mindestens einem Millimeter. Es hat sich gezeigt, dass eine solche grobe Riffelung mit Knochenzement eine zuverlässige und langzeitstabile formschlüssige Befestigung schafft.

Unter scharfkantig wird bei der geriffelten Struktur verstanden, dass vorstehende Bereiche der Riffelung ohne verrundete Kanten ausgeführt sind.

Unter einem Verankerungskiel einer Endoprothese wird eine Struktur verstanden, die zur Verankerung der Endoprothese in einem Knochen, insbesondere einem Markraum des Knochens vorgesehen ist und einen Schaft sowie seitlich daran angeordnete flügelartige Ausleger umfasst.

Unter dem "untermaßig" wird bei konturähnlichen Objekten verstanden, dass die Außenkontur des einen Objekts innerhalb der Außenkontur des anderen verläuft, und zwar vorzugsweise in den Abmessungen um ein bestimmtes absolutes oder relatives Maß verringert.

Die poröse Struktur weist vorzugsweise eine Porosität im Bereich von 60% bis 90% und/oder eine durchschnittliche Porengröße von 0,1 mm bis 1,5 mm auf, insbesondere von 0,4 mm bis 1,0 mm. Die Dicke der porösen Struktur beträgt vorzugsweise zwischen 0,6 mm und 2,5 mm, wobei die Dicke so bemessen ist, dass mindestens eine in der Tiefe des Materials (also nicht an der Oberfläche liegen bzw. angeschnitten) befindliche Schicht von Poren vorgesehen ist.

Durch die elastische Ausführung des scheibenförmigen Augments wird ermöglicht, dass dieses am Knochenkopf in einer von der Kortikalis umgebenen Aushöhlung eingesetzt und versenkt werden kann. Hierbei kommt das scheibenförmige Augment mit seiner Außenfläche zur Anlage an die Innenseite der Kortikalis, und durch die beim Einsetzen vorgenommene Kompression der Schenkel wird die poröse Struktur am Außenmantel gegen die Innenseite der Kortikalis gepresst. Damit wird eine innige Verbindung zwischen der porösen Struktur einerseits und der Kortikalis andererseits geschaffen, was eine günstige Primärfixierung und nachfolgend beste Voraussetzungen für das Einwachsen von Knochenmaterial und damit für eine entsprechend langfristig stabile Befestigung schafft. Durch die Deckplatte wird oben auf das Augment aufgebrachter Knochenzement separiert von dem inneren und unteren Bereich des Augments, sodass dort keine störende Fixierung durch Knochenzement auftritt und außerdem wird das Einwachsen von Knochenmaterial nicht durch eindringenden Zement gestört. Dies wiederum schafft die Möglichkeit, die Verbindung zwischen dem Augment und der proximalen darüber liegenden Endoprothese, beispielsweise die Tibiaplatte einer Kniegelenkendoprothese, mittels eines Zementbetts zu schaffen. Dies ergibt dank der Riffelung an der proximalen Seite des erfindungsgemäßen Augments einen guten Halt des Zementbetts und damit auch der proximalen angeordneten Endoprothese. Damit schafft die Deckplatte weiter die Voraussetzung dafür, mittels des aufgebrachten Zementbetts eine Durchschneideschicht zu schaffen, welche im Fall einer geplanten Explantation auf verhältnismäßig einfache Weise mittels einer Säge zu durchtrennen ist, wodurch die Endoprothese freikommt von dem Augment und somit leicht entnommen werden kann, ohne das ggf. stark in den Knochenkopf eingewachsene Augment herauszureißen.

Es ist das Verdienst der Erfindung, eine eigenständige Befestigung des Augments mittels des poröse Außenmantels, der durch mit Kompression aufgebrachter Vorspannkraft gegen die Kortikalis am Knochenkopf gedrückt ist und somit optimale Voraussetzung zum Knocheneinwuchs schafft, zu kombinieren mit einer Deckplatte, welche die Aufbringung einer Zementschicht ermöglicht, die dank der Riffelung am Augment fixiert ist und so die darüber liegende Endoprothese befestigt, und zusätzlich am Ende der Lebensdauer noch als Durchschneideschicht fungiert für ein einfaches Abtrennen der Endoprothese von dem im Knochen eingewachsene Augment, und zwar ohne Knochenschädigung. Somit verknüpft die die Erfindung gegensätzlich erscheinende Vorteile in Bezug auf Befestigungssicherheit einerseits und schadlosen Explantation andererseits. Revisionsoperationen mit Austausch der Endoprothese führen so zu einer Verringerung des Risikos für den Patienten.

Vorzugsweise ist die distale Seite des scheibenförmigen Augments als poröse Struktur ausgeführt. Damit werden an der gro-ßen Kontaktfläche, mit der die distale Seite auf dem Knochen flächig anliegt, günstige Bedingungen für Knocheneinwuchs und somit gute Befestigung des Augments geschaffen. Mit Vorteil sind die Kanten massiv ausgeführt. Unter Kanten werden die Übergänge zwischen Seitenflächen verstanden, insbesondere von der distalen Seite zu lateralen Seiten. Damit wird eine Verstärkung der Kanten erreicht, die ein Ausbrechen der porösen Struktur verhindern.

Mit Vorteil ist die poröse Struktur so ausgeführt, dass sie in der Tiefe des Materials verbundene Poren umfasst. Die Porosität geht somit über eine oberflächliche Porosität hinaus und reicht bis in die Tiefe des Materials. Es werden so Hohlräume geschaffen, die miteinander in Verbindung stehen, wodurch besonders günstige Bedingungen für den Einwuchs von Knochenmaterial geschaffen sind. Mit Vorteil sind die Poren so dimensioniert, dass sie eine Weite von etwa 0,4 mm bis 1,0 mm aufweisende. Damit ergibt sich ein besonders günstiges Einwuchsverhalten. Zweckmäßig ist, wenn die poröse Struktur in der Tiefe mindestens eine und bis zu drei Schichten von Poren umfasst, was typischerweise eine bevorzugten Dicke der porösen Struktur von etwa 0,6 mm bis 2,5 mm bedeutet.

Zweckmäßigerweise ist vorgesehen, dass die poröse Struktur poröse Bereiche aufweist, die von einem massiven Rand umrahmt sind. Damit wird zum einen eine eindeutige Begrenzung erreicht und zum anderen ein unerwünschtes Ausbrechen von mechanisch empfindlicheren porösen Material aus der poröse Struktur insbesondere im Randbereich vermieden.

Es ist möglich, dass die Schenkel aus einer porösen Struktur gebildet sind, wobei die proximale Seite selbstverständlich weiterhin wie bereits oben angegeben mit einer Deckplatte versehen ist. Dies ergibt zum einen eine leichte Struktur, die zum anderen nahezu durchgängig offen für den Eindruck von Knochenmaterial ist. Bevorzugt ist allerdings, dass die Schenkel einen massiven (nicht porösen) Kern aufweisen, an dessen Außenseiten Taschen gebildet sind, in denen die poröse Struktur angeordnet ist. Damit können definierte Zonen geschaffen werden, an denen die poröse Struktur vorhanden ist und wo ein Einwuchs des Knochenmaterials erfolgen solle. Außerdem werden so die Schenkel bzw. das Augment mechanisch robuster und belastbarer, ferner wird ein unnötig tiefes Einwachsen des Knochenmaterials verhindert. Letzteres bringt mit zunehmender Tiefe nur geringe Vorteile in Bezug auf die Befestigungssicherheit, jedoch bedeutet ein solch besonders tiefes Durchwachsen mit Knochenmaterial meist eine erhebliche Erschwernis bei einer Explantation des Augments. Durch die Anordnung der Taschen kann die Einwuchstiefe begrenzt werden, wobei wie bereits vorstehend erwähnt, die poröse Struktur mindestens eine Schicht von in der Tiefe des Materials verbunden Poren umfasst.

Mit Vorteil ist die Innenwandung an den Schenkel massiv ausgeführt. Ein Einwachsen von Knochenmaterial an dieser schwer zugänglichen Stelle ist in der Regel nicht erwünscht, zumal die Innenwandung typischerweise nur Kontakt hat mit dem Verankerungskiel und anderen Befestigungselementen, wie beispielsweise Schrauben. Das gilt auch in Bezug auf andere laterale Flächen als den Außenmantel, bspw. gilt dies auf für Innenwände, zwischen denen Schlitze oder Spalte gebildet sind.

Bei einer besonders bevorzugten Ausführungsform, die ggf. unabhängigen Schutz verdient, sind die Schenkel in Skelettbauweise ausgeführt mit mehreren durch Schlitze getrennten, nebeneinanderliegenden Scheibensegmenten, die über flexible Stege an einem Rahmen angeordnet sind. Das scheibenförmige Augment ist somit nicht als eine unitäre Scheibe ausgeführt (mit einer Durchgangsöffnung), sondern ist in mehrere nebeneinanderliegende Scheibensegmente unterteilt. Sie weisen typischerweise dieselbe Dicke auf. Die Scheibensegmente sind über flexible Stege an dem Rahmen befestigt. Damit ist eine Kompression der Schenkel ermöglicht, ohne dass es zu Verspannungen zwischen den Scheibensegmenten kommt. Die Skelettbauweise ermöglicht ein besonders günstiges Kompressions- und Elastizitätsverhalten, was das Einsetzen des Augments in einen am Knochenkopf geschaffenen Rezess vereinfacht und dort für eine erhöhte Befestigungssicherheit sorgt. Eine ausreichende Dichtwirkung gegenüber Knochenzement ist gegeben, so dass bei dieser Ausführungsform eine durchgehende Dichtplatte nicht erforderlich ist; es genügt, wenn jedes der Scheibensegmente seine eigene Deckplatte bildet. Ein weiterer Vorteil der Skelettbauweise ist, dass Augmente in verschiedenen Größenstufen durch Hinzufügen oder Weglassen von einem oder mehreren Scheibensegmenten gebildet werden können.

Vorzugsweise sind die Schlitze so eng bemessen, dass sie als Spaltdichtung für Knochenzement fungieren. Somit wird auf einfache und wenig aufwändige Weise eine ausreichende Abdichtung gegenüber Knochenzement erreicht. Vorzugsweise sind die Schlitze Vorzugsweise maximal 1 mm weit, weiter vorzugsweise maximal 0,7 mm. Je nach Höhe der Scheibensegmente können grö-ßere Weiten genügen oder es können kleinere Weiten erforderlich sein, um einen ausreichenden Dichtspalt zu erhalten.

Mit Vorteil sind die laterale Flächen der Scheibensegmente (in der Regel sind dies deren lateral Außenseiten bzw. die Schlitze bildenden Seitenflächen) massiv ausgeführt, also nicht porös. Damit wird eine Aussteifung erzielt. Weiter wird so der Gefahr eines unerwünschten Eindringens von Knochenzement in bzw. durch die Schlitze wirksam begegnet. Vorzugsweise sind die Scheibensegmente massiv ausgeführt, ggf. mit Taschen für poröse Struktur am Außenmantel und/oder des distalen Seite.

Zweckmäßig ist, wenn der Rahmen als ein die Schenkel mindestens hälftig umschließender Außenrand ausgeführt ist, an dessen Außenseite der Außenmantel angeordnet ist. Der Rahmen berandet die Gesamtheit der Scheibensegmente, welche sich somit auf der Innenseite des Rahmens befinden. Der Rahmen kann so zugleich den Außenmantel mit der porösen Struktur bilden. Die durch die Kompression sich ergebende Spannungsbelastung verteilt sich auf den Rahmen, was zu einer gleichmäßigen Belastung und damit zu einer höheren Beanspruchungsfähigkeit führt.

Vorzugsweise ist in den Schenkel mindestens ein Befestigungsloch zur Aufnahme einer Befestigungsschraube vorgesehen. Damit kann eine zusätzliche Befestigung des Augments am Knochen erreicht werden, was insbesondere einen wertvollen Beitrag zur Primärbefestigung leisten kann. Mit Vorteil weist das Befestigungsloch einen massiven Lochmantel auf. Durch eine solche Auskleidung wird verhindert, dass einwachsendes Knochenmaterial eine Verbindung zwischen Schraubengewinde und der porösen Struktur des Augments herstellt, wodurch das Lösen der Schraube erschwert würde.

Bei der Skelettbauweise ist es zweckmäßig, wenn das Befestigungsloch in einem der Scheibensegmente angeordnet ist, wobei dieses Scheibensegment nicht mittels eines schmalen Stegs am Rahmen angeordnet ist, sondern mittels einer breiteren Brücke, die mindestens doppelt so breit wie die Stege ist. Damit werden zwei Vorteile erreicht: zum einen wird eine leistungsfähige Befestigung des durch die Befestigungsschraube zusätzlich belasteten Scheibensegmente bewirkt. Zum anderen ermöglicht es die breitere Brücke, das Befestigungsloch näher an den Rand damit in die Nähe des Rahmens zu rücken, was insbesondere bei kleinen Größen des Augments durch die so verbesserte Raumausnutzung ein erheblicher Vorteil ist.

Die Durchgangsöffnung ist typischerweise ein langgestreckter Aufnahmeraum für einen Verankerungskiel der Endoprothese. Die Durchgangsöffnung kann zweckmäßigerweise zu einer Seite hin offen sein. Dies ist besonders dann ein Vorteil, wenn das Augment nur halbseitig vorgesehen ist. Der Begriff halbseitig bezieht sich hierbei auf die Abmessungen der Endoprothese, deren Verankerungskiel durch die Durchgangsöffnung des Augments geführt ist. Die Endoprothese benötigt häufig nur auf einer Seite Unterfütterung, beispielsweise wenn ein Knochendefekt auf eine Seite des Knochenkopfs beschränkt ist und das Augment daher nur dort benötigt wird. Es versteht sich, dass auch zwei halbseitige Augmente vorgesehen sein können, um beide Seiten zu unterfüttern.

Die Durchgangsöffnung ist typischerweise eher klein bezogen auf die Abmessungen des Augments, in der Regel nimmt sie weniger als die Hälfte, häufig weniger als ein Drittel des Volumens des Augments ein. Dies hat den Vorteil, dass somit noch ausreichend stützendes Material für die Schenkel bzw. die Scheibensegmente verbleibt, um eine ausreichende Unterfütterung zu gewährleisten. Sie ist in der Regel so bemessen, dass sie mit geringem Spiel den Verankerungskiel ggf. dessen flügelartige Ausleger aufnimmt. Das geringe Spiel ist zweckmäßigerweise so gewählt, dass eine ausreichende Dichtwirkung gegenüber Knochenzement besteht.

Der Außenmantel steht mit Vorteil nicht in einem 90° Winkel zur proximalen Seite bzw. zur distalen Seite. Es hat sich bewährt, wenn der Außenmantel konisch geneigt ist, vorzugsweise mit einem Winkel von 5 bis 10° nach distal verjüngend. Dank dieser Konizität kann das Augment leichter durch Zusammendrücken eingesetzt bzw. entnommen werden, als dies bei senkrecht stehendem Außenmantel der Fall wäre.

Zweckmäßigerweise sind an dem Außenmantel nach außen gerichtete Spikes angeordnet. Sie pressen sich im implantierten Zustand in die umlaufende Kortikalis des Knochenkopfes, in welchem das Augment in einem Rezess aufgenommen ist. Damit wird einer Erhöhung der Befestigungssicherheit erreicht, und zwar bereits von Beginn an, also eine verbesserte Primärfixierung. Beim Zusammendrücken (Kompression) der Schenkel des Augments zur Explantation bewegen sich die Spikes dann selbsttätig zurück aus der Kortikalis, wodurch sie die Explantation freigeben.

Mit Vorteil ist die geriffelte Struktur genutet ausgeführt. Zweckmäßigerweise sind durchlaufende Nuten vorgesehen, die vorzugsweise in eine Richtung im wesentlichen quer zur Erstreckung der Durchgangsöffnung orientiert sind. Durch die Nutung wird eine besonders wirksame Verzahnung mit einem aufgebrachten Zementbett erreicht. Die Nutung weist vorzugsweise solche Abstände und Höhe auf, die auf das Fließ- und Abbindeverhalten sowie die Körnung des Zementbetts abgestimmt ist. Bewährt haben sich Nut-Abstände im Bereich von 2 bis 10 mm und/oder einer Nut-Tiefe von 0,5 bis 2 mm, wobei die Nuten begrenzende Profile etwa 0,5 bis 3 mm breit sind.

Die geriffelte Struktur kann scharfkantig ausgeführt sein. Jedoch sind auch andere alternative Profilformen denkbar, hilfsweise dreieckige oder Profilformen mit Verrundungen an den Ecken.

Zweckmäßig ist, wenn die geriffelte Struktur durch ein Drahterodier-Verfahren hergestellt ist. Damit können auf effiziente Weise nahezu beliebige Profilformen, darunter auch scharfkantige, effizient gebildet sein.

Besonders zweckmäßig ist es, wenn das Augment einheitlich durch ein additives Verfahren, insbesondere 3D-Druck, hergestellt ist. Damit können auch komplexe Formen in verschiedenen Größen ohne oder lediglich mit einem Minimum an aufwendiger Nachbearbeitung erzeugt werden. Besonders zweckmäßig ist eine Kombination mit dem Drahterodieren zum Schaffen definierter, scharf begrenzter Profile, wie vorstehend erwähnt.

Die Deckplatte kann zementdicht ausgeführt sein. Unter zementdicht wird verstanden, dass kein Knochenzement durch die Deckplatte tritt. Dazu kann die Deckplatte durchgehend ausgeführt sein oder mit insbesondere schlitzartigen Durchbrechungen versehen sein, die als Dichtungen, insbesondere Spaltdichtungen, gegen Durchtritt von Knochenzement wirken.

Mit Vorteil ist vorgesehen, dass die poröse Struktur mit einer knocheneinwuchsfördernden Beschichtung, insbesondere umfassend Kalziumphosphat, versehen ist, und/oder an nicht-porösen Bereichen mit einer bioziden Beschichtung, insbesondere umfassend Silber, versehen ist. Damit wird erreicht, dass an gewünschten Stellen das Einwachsen von Knochenmaterial begünstigt wird und an anderen Stellen, an denen dies insbesondere zum Zweck einer einfacheren Explantation nicht gewünscht ist, dies nicht geschieht.

Die Erfindung erstreckt sich ferner auf eine Anordnung aus einer Endoprothese, insbesondere einer Kniegelenkendoprothese, und dem scheibenförmigen Augment. Ein Verankerungskiel der Endoprothese ist in die Durchgangsöffnung aufgenommen, und zwar insbesondere im implantierten Zustand. Zweckmäßigerweise ist das scheibenförmige Augment in seiner Außenkontur ähnlich der Endoprothese gestaltet, insbesondere einer Tibiaplatte. Jedoch ist es gegenüber dieser untermaßig, d. h. es weist etwas kleinere Abmessungen auf (insbesondere im Bereich von 2 bis 6 mm entlang des Außenmantels). So kann es die Endoprothese, insbesondere Tibiaplatte, unterstützen, aber dennoch in einem von der Kortikalis berandeten Rezess am Knochenkopf aufgenommen sein.

Mit Vorteil ist das scheibenförmige Augment zur Unterstützung einer linken oder rechten Hälfte der Endoprothese halbseitig ausgeführt. Damit kann eine wirksame Unterstützung durch das Augment erreicht werden, wenn ein Knochendefekt nur einseitig am Knochenkopf vorliegt, und so das unnötige Entfernen von Knochenmaterial an der anderen, gesunden Seite vermieden werden.

Zweckmäßigerweise ist die Durchgangsöffnung zur Aufnahme von dem Verankerungskiel und dessen flügelartigen Auslegern mit einem definierten Freiraum von vorzugsweise 1 bis 3 mm bemessen. Mit diesem Freiraum entsteht ein Spiel zwischen dem Verankerungskiel mit dessen flügelartigen Auslegern und den Schenkeln des Augments, wodurch ein Toleranzausgleich ermöglicht ist und ein unnötiger unmittelbarer Kontakt vermieden werden kann. Für die Praxis ist es eine erhebliche Erleichterung, da so eine unerwünschte Verklemmung von Augment und Verankerungskiel mit seinen flügelartigen Auslegern vermieden werden kann.

Die Erfindung wird nachfolgend unter Bezugnahme auf die beigefügte Zeichnung anhand vorteilhafter Ausführungsbeispiele erläutert. Es zeigen:
- Fig. 1: eine perspektivische Ansicht eines ersten Ausführungsbeispiels für ein scheibenförmiges Augment;
- Fig. 2A, B: eine Rückansicht sowie eine Schnittansicht von medial des Augments gemäß Fig. 1;
- Fig. 3: eine perspektivische distale Ansicht darstellend Außenmantel und distale Seite des Augments;
- Fig. 4: verschiedene Profilformen für eine Riffelung;
- Fig. 5: eine Detailansicht darstellend porös ausgeführte Schenkel gemäß einer Variante des Augments;
- Fig. 6: eine Frontalansicht des Augments zusammen mit einer Tibiaplatte einer Kniegelenkendoprothese;
- Fig. 7: eine vergrößerte Detailansicht zur Anordnung des Augments an der Kniegelenkendoprothese;
- Fig. 8: eine Detailansicht des Augments mit eingesetztem Verankerungskiel der Kniegelenkendoprothese;
- Fig. 9: eine perspektivische distale Schrägansicht eines zweiten Ausführungsbeispiels des Augments;
- Fig. 10: eine Explosionsansicht des Augments gemäß Fig. 9 mit der Kniegelenkendoprothese;
- Fig. 11A, B: Aufsichten auf eine distale Seite des Augments in zwei verschiedenen Größen; und
- Fig. 12A, B: das Augment mit verschiedenen Tibiaplatten einer modularen Kniegelenkendoprothese.

Die Erfindung wird nachfolgend erläutert anhand eines Beispiels für ein Augment für eine Kniegelenkendoprothese, und zwar genauer gesagt für ein am oberen (proximalen) Knochenkopf der Tibia angeordnetes Augment. Das Augment ist zu unterscheiden von der Prothese, d.h. das Augment ist kein Element der eigentlichen Prothese. Das Augment verstärkt den Knochen und erhöht bzw. verbessert so dessen Aufnahmefähigkeit für die Prothese. Erfindungsgemäße Augmente können selbstverständlich auch an anderen Knochen vorgesehen sein. Das Augment 1 besteht vorzugsweise aus einer Titanlegierung, und kann zweckmäßigerweise durch ein additives Verfahren (3D-Druck) hergestellt sein. Es kann aber auch aus anderem biokompatiblen Material bestehen, beispielsweise aus metallischem Material wie bspw. Kobalt-Chrom-Molybdän (CoCrMo) oder auch aus Kunststoffmaterial, wie bspw. Polyetheretherketon (PEEK).

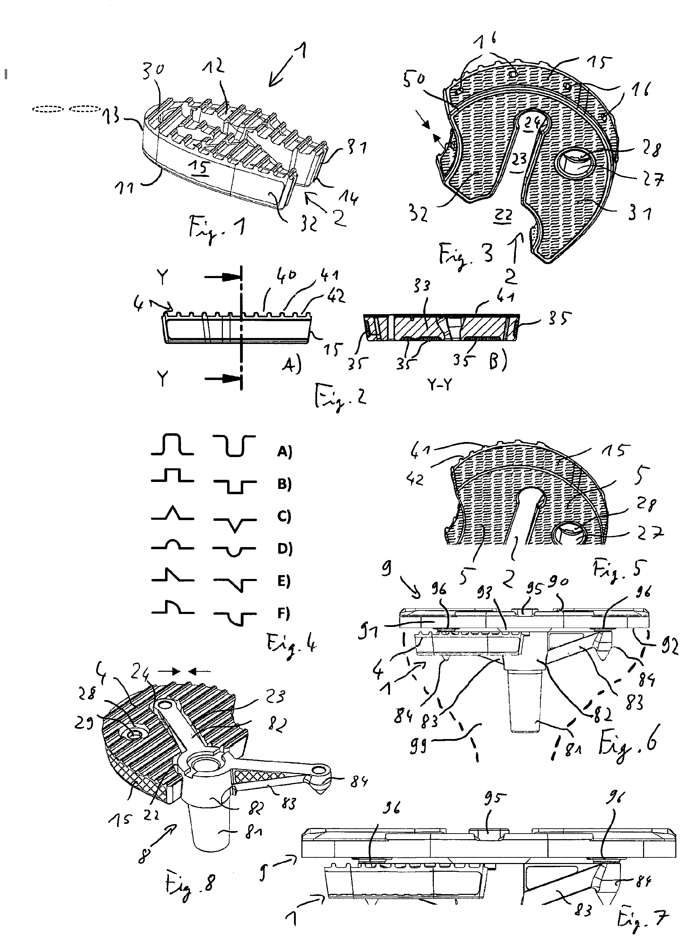

Das Augment 1 ist dazu vorgesehen, unterhalb der Tibiakomponente 9 einer Kniegelenkendoprothese angeordnet zu sein, wie sie in Figur 6 dargestellt ist. Die Kniegelenkendoprothese umfasst eine am Femurknochen (nicht dargestellt) anzuordnende femorale Komponente (nicht dargestellt) sowie die am proximalen Ende eines Tibiaknochens 99 anzuordnen Tibiakomponente 9. Diese umfasst eine Tibiaplatte 91, die lateral ausgedehnt auf einem resezierten Knochenkopf der Tibia angeordnet ist. Auf der proximalen Seite (Oberseite) der Tibiaplatte 91 ist eine Aufnahme 90 vorgesehen, in welche ein Lagerstück (nicht dargestellt) der Kniegelenkendoprothese anzuordnen ist. Die gegenüberliegende distale Seite 92 der Tibiaplatte 91 ist ausgebildet zur Anlage an der Oberfläche des Knochenkopfs der Tibia 99. Je nachdem ob die Tibiakomponente zur zementierten oder zementfreien Implantation vorgesehen ist, ist die Tibiaplatte 91 an ihrer distalen Seite 92 optional mit einer porösen Struktur zum Einwuchs von Knochenmaterial versehen, um so bei zementfreier Implantation eine optimale Befestigung zu ermöglichen.

Zur Befestigung der Tibiaplatte 91 ist ein Verankerungskiel 8 vorgesehen, welcher nach distal abstrakte. Er weist ein Schaftstück 82 und ein sich daran distal anschließendes Konusstück 81 auf. Das Konusstück 81 ist dazu ausgebildet, ggf. einen in den Markkanal der Tibia ragenden Steckschaft aufzunehmen. Nach lateral schließen sich an das Schaftstück 82 jeweils ein flügelartiger Ausleger 83 sowohl zur linken wie zur rechten Seite an, an dessen freiem Ende eine Spannhülse 84 angeordnet ist. Die flügelartigen Ausleger 83 fungieren als Tragarme für die Tibiaplatte 91. Die Tibiaplatte 91 ist somit an drei Punkten mit dem Verankerungskiel 8 verbunden, nämlich zentral an dem Schaftstück 82 und jeweils links- und rechtsseitig mit den an freien Enden der flügelartigen Ausleger 83 angeordneten Spannhülsen 84. Dazu sind an der Tibiaplatte 91 zwei Verschraubungslöcher 94 links und rechts vorgesehen, die mit der jeweiligen Spannhülse 84 fluchten.

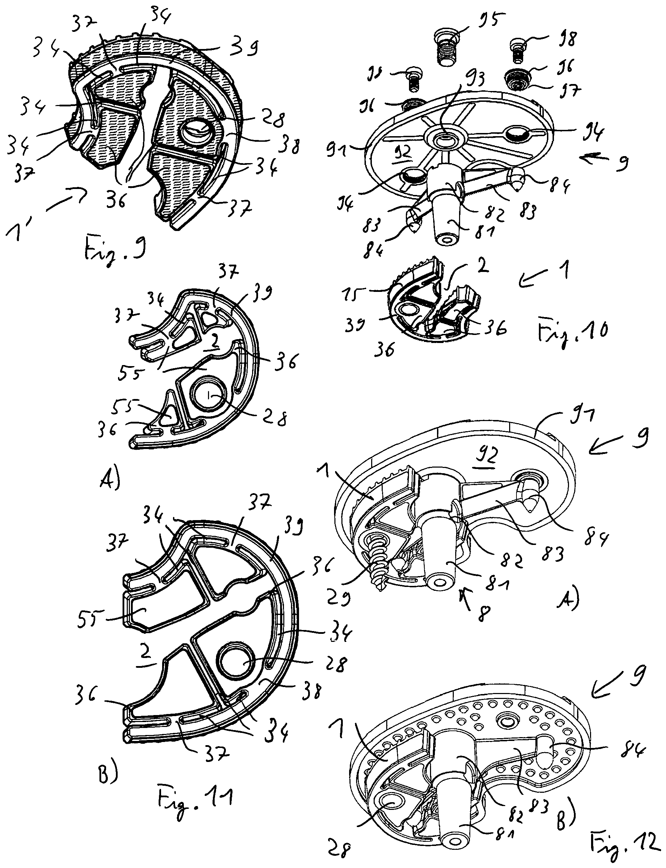

Hierbei liegen die flügelartigen Ausleger 83 nicht bündig an der Unterseite 92 an, sondern sind mit einem definierten Abstand gehaltert. Dazu ist an der Unterseite 92 ein zentraler Auflagebund 93 vorgesehen, an welchem das Schaftstück 82 zur Anlage kommt. In die Verschraubungslöcher 94 sind Abstandseinsätze 96 mit einem Außengewinde 97 eingeschraubt (s. Fig. 10), welche einen definierten Abstand zu den Spannhülsen 84 herstellen. Auf diese Weise wird zusammen mit dem zentralen Auflagebund 93 ein definierter Abstand zwischen der Unterseite 92 der Tibiaplatte 91 und den flügelartigen Auslegern 83 des Verankerungskiels 8 eingestellt. Dies ist in Figur 6 dargestellt, und der definiert eingestellte Abstand ist in der Detaildarstellung gemäß Figur 7 durch die mit gestrichelter Linie dargestellten Ellipsen hervorgehoben.

Figur 1 zeigt eine perspektivische Ansicht eines ersten Ausführungsbeispiels für ein Augment 1 gemäß der vorliegenden Erfindung. Das Augment 1 ist wie eine dicke Scheibe geformt und weist zwei Schenkel 31, 32 auf, die über ein Verbindungsstück 30 am gemeinsamen Ende der Schenkel 31, 32 gelenkartig elastisch gekoppelt sind. Zwischen den Schenkeln 31, 32 ist eine Durchgangsöffnung 2 angeordnet, die zur Aufnahme eines Verankerungskiels 8 der Tibiakomponente 9 ausgebildet ist.

Eine Rückansicht des Augments 1 sowie eine Schnittansicht von medial sind den Figuren 2A und B dargestellt. Die Rückansicht bezieht sich hierbei auf die Orientierung des Augments 1 im eingesetzten Zustand, also unter Nutzung anatomischer Fachausdrücke handelt es sich um eine Ansicht von posterior. Oben, also auf der proximalen Seite, auf dem Augment 1 ist eine zementdichte Deckplatte 4 ausgebildet. Diese ist auf ihrer Oberseite mit einer geriffelten Struktur 40 versehen ist. Die geriffelte Struktur 40 dient als Verzahnung zu einem Zementbett (nicht dargestellt), welches den Zwischenraum zu der Tibiaplatte 91 auffüllt, und sorgt so für eine zusätzliche Befestigung. Die Riffelung 40 weist eine Nutung 42 auf, wobei die einzelnen Nuten durch erhabene Profile voneinander abgegrenzt sind. Die Nutung ist in eine Richtung von vorne nach hinten orientiert, also bezogen auf den implantierten Zustand von anterior nach posterior. Die Tiefe der Nutung 40 beträgt beispielsweise 1 mm, die Breite der Nutung etwa 6 mm und die Breite der erhabenen Profile 41 beträgt etwa 1,5 mm. Im einfachsten Fall können die Profile 41 eine rechteckige Querschnittsform aufweisen, wie sie in Figur 5A) oder 5B) dargestellt sind, mit verrundeten bzw. scharfen Ecken, jeweils als positives oder negatives Profil. Alternativ sind auch nicht rechteckige Formen für das Profil möglich, beispielsweise in Form eines symmetrischen Dreiecks oder eines asymmetrischen, sägezahn-artigen Dreiecks, wie in Figur 5C) bzw. 5E) dargestellt. Ferner können auch verrundete Profile vorgesehen sein, beispielsweise nach Art eines Halbrund wie in Figur 5D) oder asymmetrisch nach Art eines Viertelkreises wie in Figur 5F) dargestellt. Dies kann der Fachmann je nach Anforderungen an die durch die Profile 41 gebildete Verzahnung mit dem Knochenzement auswählen.

In Figur 2A, B erkennt man ferner an den jeweiligen lateralen Seiten des Augments 1, dass diese konisch nach distal (in der Abbildung nach unten) zulaufend sind. In dem dargestellten Ausführungsbeispiel beträgt der Winkel auf jeder Seite etwa 7°. Es können auch andere Winkel vorgesehen sein. Die konische Gestaltung vereinfacht nicht nur das Einsetzen bei der Implantation, sondern vor allem auch ein Entnehmen des Augments 1 nach oben im Rahmen einer Explantation.

Es wird nun Bezug genommen auf die Figuren 2B) sowie Figur 3. Wie in Figur 3 durch strichlierte Darstellung visualisiert ist, sind die distale Unterseite 11 des Augments 1 sowie ein Außenmantel 15 an der Lateralseite 13 jeweils mit einer porösen Struktur 5 versehen. Bei der dargestellten Ausführungsform geht die poröse Struktur nicht durch, sondern die Schenkel 31, 32 weisen einen massiven Kern 33 auf. Es sind Taschen 35 an den entsprechenden Oberflächen der Schenkel 31, 32 vorgesehen, in denen die poröse Struktur 5 angeordnet ist. Die Taschen 35 weisen eine Tiefe von etwa 0,8 bis 1,5 mm auf. In Figur 2B sind derartige Taschen an der distalen Seite 11 sowie lateral an dem Außenmantel 15 vorgesehen und dargestellt. Um ein unerwünschtes Ausbrechen der porösen Struktur 5 insbesondere an den Rändern Kanten des Augments 1 zu verhindern, sind die Kanten zwischen benachbarten porösen Strukturen 5 am Übergang von der distalen Seite zur Lateralseite mit massiv, also nicht porös ausgeführten Kanten 50 als Rahmen versehen. Somit wird dort eine Verstärkung erreicht und ein Ausbrechen der porösen Struktur 5 verhindert. Die Breite dieser Kanten 50 ist durch die beiden gegenläufigen Pfeile in Figur 2 visualisiert und beträgt vorzugsweise etwa 1mm bis 2 mm.

Ferner können an dem Außenmantel 15 nach außen weisende Spikes 16 angeordnet sein. Diese bohren sich nach der Implantation des Augments 1 in die umlaufende Kortikalis des Tibiakopfs und sichern somit das Augment 1 zusätzlich in seiner Position. Ferner ist an dem Schenkel 31 ein Befestigungsloch 38 vorgesehen. Es ist so bemessen, dass eine Spongiosaschraube 29 zusätzliche Befestigung diese Öffnung gesteckt und angezogen werden kann. Zur zusätzlichen Aussteifung ist das Befestigungsloch 28 mit einem massiven Lochmantel 27 als Innenauskleidung versehen.

Wie man weiter aus Figur 3 gut erkennen kann, ist die Durchgangsöffnung 2 in mehrere Bereiche gegliedert. Den größten Bereich nimmt am Rand der Durchgangsöffnung ein Aufnahmebereich 22 für den das Schaftstück 82 des Verankerungsteil 8 ein, an den sich ein lang gestreckter Bereich 23 der Durchgangsöffnung erstreckt, welcher zur Aufnahme des flügelartigen Auslegers 83 ausgebildet ist. An dem fernen Ende hiervon ist ein kreisrunder Aufnahmebereich 24 die für die Spannhülsen 84 am freien Ende des jeweiligen flügelartigen Auslegers 83 vorgesehen. Wie insbesondere durch ein Vergleich mit Figur 8 erkannt werden kann, ist die Durchgangsöffnung 2 mit ihren Bereichen 22, 23 und 24 auf das Verankerungsteil 8 bezüglich der Dimensionierung abgestimmt. Somit ergibt sich eine Aufnahme des Verankerungsteil 8 mit Spiel, jedoch ist das Spiel verhältnismäßig gering beträgt bei dem in Figur 8 dargestellten Beispiel etwa 1,5 mm, wobei dieser Spielabstand durch die beiden gegenläufigen Pfeile in Figur 8 visualisiert ist. Durch dieses geringe Spiel entsteht so die Wirkung einer Spaltdichtung zum Verankerungskiel 8.

Figur 5 zeigt eine Variante des in Figur 3 dargestellten Augments. Anders als in Figur 3 ist nun kein massiver Kern 33 für die Schenkel 31, 32 vorgesehen, sondern diese sind durchgängig aus poröser Struktur 5 gebildet, bis auf die Deckplatte 4 an der proximalen Seite 12. Diese Ausführung ist sehr leicht und ermöglicht ein gutes Einwachsen von Knochenmaterial rundum. Hierbei sind die verbreiterten Kanten 30 zum Schutz der porösen Struktur 5 gerade in dem kritischen Kantenbereich nicht mehr vorhanden, sodass eine erhöhte Gefahr des Ausbrechen von Bestandteilen aus der porösen Struktur 5 besteht.

Ein zweites Ausführungsbeispiel des Augments ist als eine perspektivische distale Schrägansicht in Figur 9 dargestellt. Gleichartige Elemente wie bei dem ersten Ausführungsbeispiel tragen gleiche Bezugszeichen, wobei unterschiedliche Komponenten verschiedene Bezugsziffer tragen. Bei dieser Ausführungsform ist das scheibenförmige Augment 1' in Skelettbauweise ausgeführt. Es weist dazu einen mindestens Dreiviertel des Umfangs des Augments 1' umlaufenden Rahmen 39 auf. An diesem sind in dem dargestellten Ausführungsbeispiel jeweils innenseitig eine Mehrzahl von Scheibensegmenten 36 angeordnet, wobei zwischen den Scheibensegmenten 36 die Durchgangsöffnung 2 gebildet ist. Die Durchgangsöffnung 2 mit ihren verschiedenen Bereichen 22, 23 und 24 ist so gestaltet wie bei dem ersten Ausführungsbeispiel, worauf hiermit zur Vermeidung unnötiger Wiederholungen verwiesen wird.

Die Scheibensegmente 36 sind jeweils über einen Steg 37 elastisch innen an dem Rahmen 39 angeordnet. Somit sind zwischen den Scheibensegmenten 36 sowie zwischen Scheibensegmenten 36 und der Innenseite Wandung des Rahmens 39 schmale Schlitze 34 gebildet, die Freiraum für eine relative Bewegung der Scheibensegmente bei einer Kompression/Expansion bieten. Die Weite der Schlitze 34 ist so bemessen, dass unter Berücksichtigung der durch die Dicke des Augments 1' vorgegebenen Tiefe der Schlitze 34 eine gegen den Eintritt von Knochenzement wirksame Spaltdichtung gebildet ist. Auf diese Weise kann die Flexibilität der über jeweils einen der Stege 37 flexibel angeordneten Scheibensegmente 36 kombiniert werden mit einer das Eindringen von Knochenzement auf so einfache wie robuste Weise vermeidenden Konstruktion. Mit diesen Scheibensegmenten 36 kann eine feine Anpassung an den Raumbedarf der Durchgangsöffnung 2 erfolgen, welcher wiederum von der Beschaffenheit des Verankerungskiels 8 der zu implantieren in Prothese maßgeblich bestimmt ist.

Ferner ermöglichen es die Scheibensegmente 36, auf einfache Weise Varianten des Augments in größeren oder kleineren Größen bereitzustellen. Indem beispielsweise eines der Scheibensegmente 96 weggelassen wird, kann eine kleinere Größe des Augments 1 problemlos gebildet werden, wie es in Figur 11A) dargestellt ist, oder durch Hinzufügen eines Scheibensegments eine größere Größe, wie in Figur 11B) dargestellt. Auf diese Weise ergeben sich Synergie-Effekte bei Bereitstellung von erfindungsgemäßen Augmenten in einem Augment-Satz mit verschiedenen Größen.

Die poröse Struktur 5 kann mit einer biokompatiblen Beschichtung 55 versehen sein, beispielsweise aus Kalziumphosphat zur weiteren Förderung von Knocheneinwuchs. Dies gilt für die poröse Struktur 5 aller Ausführungsformen.

In eine der Schalensegmente 36 kann das Befestigungsloch 28 angeordnet sein. Wie auch bei der vorstehend beschriebenen ersten Ausführungsform ist das Befestigungsloch 28 zweckmäßigerweise mit einem Lochmantel 27 als Innenauskleidung versehen. Ferner ermöglicht es die Erfindung, das Befestigungsloch 28 näher an den Rand des Scheibensegments zu bringen, und zwar vorzugsweise in die Nähe des umlaufenden Rahmens 39. Um dies zu ermöglichen, ist zweckmäßigerweise der Steg an dieser Stelle deutlich verbreitert zu einer Brücke 38, welche zusätzliche Raum bietet um so das Befestigungsloch 28 möglichst dicht an dem Rahmen 39 anzuordnen. Auf diese Weise wird eine gleichmäßige Anordnung des Befestigungslochs 28 und der daran angeordneten Spongiosaschraube 29 über die bei verschiedenen Prothesengrößen hinweg erreicht.

Eine Explosionsdarstellung in Bezug auf das zweite Ausführungsbeispiels ist in Figur 10 dargestellt. Man erkennt als Hauptkomponenten das Augment 1 und die Tibiaplatte 91 mit ihrem Verankerungskiel 8. Wie bereits vorstehend beschrieben, erfolgt eine Montage bzw. Zusammenbau, indem das Verankerungsteil 8 mit einem Schaftstück 82 mittels der Zentralschraube 95 unten an der distalen Seite 11 der Tibiaplatte 91 befestigt ist. Zusätzlich sind Abstandseinsätze 96 aus Titanmaterial mit einem Außengewinde 97 in die Verschraubungslöcher 94 eingeschraubt und bestimmen so einen definierten Abstand zwischen den flügelartigen Auslegern 83 und der distalen Seite 92 der Tibiaplatte 91. Zur zusätzlichen Befestigung und Abstützung sind weiter Befestigungsschrauben 98 vorgesehen, welche durch den jeweiligen Abstandseinsatz 96 durchgeführt sind und die in ein Innengewinde der jeweiligen Spannhülse 84 eingreifen und diese somit gegen den Abstandseinsatz 96 spannen. Auf diese Weise wird ein definierter Abstand zwischen den flügelartigen Auslegern 83 und der distalen Seite 92 der Tibiaplatte 91 eingestellt.

Mit diesem Abstand wird Raum geschaffen für einen Sägeschlitz, in den bei einer Explantation eine Säge eingeführt werden kann. Dieser Sägeschlitz ist in Figur 7 dargestellt (siehe insbesondere den durch die gestrichelte Ellipse markierten Bereich). Zur Explantation werden die Befestigungsschrauben 98 herausgeschraubt und die Abstandseinsätze 96 ebenfalls entnommen. Nunmehr ist der Sägeschlitz von der Seite her bis zum Schaftstück 82 in der Mitte der Tibiaplatte 91 zugänglich. Die Unterseite der Tibiaplatte 91 kann somit mittels einer Säge (nicht dargestellt) frei geschnitten werden. Schließlich wird die zentrale Schraube 95 entfernt, und die Tibiaplatte 91 kann nunmehr abgenommen werden. Auf diese Weise wird das Augment 1 mit einer ggf. darauf angeordneten Zementschicht zugänglich, welche dann bei Bedarf ebenfalls entfernt werden kann. Gewünschtenfalls kann bei Bedarf das Augment 1 durch Zusammendrücken der Schenkel 31, 32 nach oben explantiert werden.

Das erfindungsgemäße Augment 1 eignet sich insbesondere zur Anwendung bei einer modularen Kniegelenkendoprothese. Beispiele hierfür sind Figur 12A, B dargestellt. Das jeweilige Augment ist angeordnet auf einer Seite des Verankerungskiels 8, wobei jeweils unterschiedliche Tibiaplatten 91 vorgesehen sein können. In Figur 12A ist eine Tibiaplatte 91 mit einer porösen distalen Seite 92 vorgesehen, während in Figur 12B eine anders gestalteten Tibiaplatte 91' vorgesehen ist. Ferner ist in Figur 12A gezeigt, dass das Augment 1 mittels einer Spongiosaschraube 29 zusätzlich befestigt sein kann. Es versteht sich, dass eine solche Spongiosaschraube, die in das Befestigungsloch 28 aufgenommen ist, auch bei den anderen dargestellten Ausführungsformen vorgesehen sein kann.

## Patentansprüche

1. Scheibenförmiges Augment zum Ausfüllen von Knochendefekten, insbesondere am Ende von langen Röhrenknochen wie Tibia, mit einer proximalen Seite (12), einer distalen Seite (11), einem Außenmantel (15) an Lateralseiten sowie einer Innenwandung für eine von der proximalen zur distalen Seite laufende Durchgangsöffnung (2) für einen Verankerungskiel (8) einer an der proximalen Seite angeordneten Endoprothese (9), wobei das Augment eine generell C-förmige Gestalt mit zwei die Durchgangsöffnung flankierenden Schenkeln (31, 32) aufweist,
**dadurch gekennzeichnet, dass**
ein Verbindungsstück (30) zwischen den Schenkeln (31, 32) gelenkartig ausgeführt ist und mit den Schenkeln (31, 32) elastisch zusammenwirkt, so dass bei Kompression der Schenkel (31, 32) eine nach außen gerichtete Rückstellkraft erzeugt ist, und
der Außenmantel (15) als eine Knocheneinwuchs fördernde poröse Struktur (5) ausgeführt ist sowie
die proximale Seite (12) mit einer Deckplatte (4) versehen ist, die eine geriffelte Struktur (40) als Verzahnung für eine Zementverbindung aufweist.

2. Scheibenförmiges Augment nach Anspruch 1, **dadurch gekennzeichnet, dass** die distale Seite (11) als poröse Struktur (5) ausgeführt ist, wobei vorzugsweise Kanten (50) massiv ausgeführt sind.

3. Scheibenförmiges Augment nach Anspruch 1 oder 2, **dadurch gekennzeichnet, dass** die poröse Struktur (5) miteinander in der Tiefe des Materials verbundenen Poren umfasst, die vorzugsweise eine Weite von 0,4 bis 1,0 mm aufweisen.

4. Scheibenförmiges Augment nach einem der vorangehenden Ansprüche, **dadurch gekennzeichnet, dass** die poröse Struktur (5) poröse Bereiche aufweist, die von einem massiven Rand umrahmt sind.

5. Scheibenförmiges Augment nach einem der vorangehenden Ansprüche, **dadurch gekennzeichnet, dass** die Schenkel (31, 32) einen massiven Kern (33) aufweisen, an dessen Außenseite Taschen (35) gebildet sind, in denen die poröse Struktur (5) angeordnet ist.

6. Scheibenförmiges Augment nach einem der Ansprüche 1 bis 4, **dadurch gekennzeichnet, dass** die Schenkel (31, 32) aus einer porösen Struktur (5) gebildet sind.

7. Scheibenförmiges Augment nach einem der vorangehenden Ansprüche, **dadurch gekennzeichnet, dass** Innenwandungen an den Schenkeln (31, 32) massiv ausgeführt ist.

8. Scheibenförmiges Augment nach einem der vorangehenden Ansprüche, **dadurch gekennzeichnet, dass** die Schenkel (31, 32) in Skelettbauweise ausgeführt sind mit mehreren durch Schlitze (34) getrennten, nebeneinanderliegenden Scheibensegmenten (36), die über flexible Stege (37) an einem Rahmen (39) angeordnet sind, wobei vorzugsweise der Rahmen (39) als ein die Schenkel (31, 32) mindestens hälftig umschließender Außenrand ausgeführt ist, an dessen Außenseite der Außenmantel (15) angeordnet ist.

9. Scheibenförmiges Augment nach Anspruch 7 oder 8, **dadurch gekennzeichnet, dass** die Schlitze (34) so eng bemessen sind, dass sie als Spaltdichtung für Knochenzement fungieren, vorzugsweise maximal 1 mm weit sind, weiter vorzugsweise maximal 0,7 mm.

10. Scheibenförmiges Augment nach einem der vorangehenden Ansprüche, **dadurch gekennzeichnet, dass** in den Schenkeln (31, 32) mindestens ein Befestigungsloch (28) zur Aufnahme einer Befestigungsschraube (29) vorgesehen ist, wobei das Befestigungsloch (27) vorzugsweise einen massiven Lochmantel (27) aufweist.

11. Scheibenförmiges Augment nach Anspruch 10, **dadurch gekennzeichnet, dass** das Befestigungsloch (28) in einem der Scheibensegmente (36) nach Anspruch 7, 8 oder 9 angeordnet ist, wobei dieses Scheibensegment (36) mittels einer gegenüber den Stegen (37) mindestens doppelt so breiten Brücke (38) am Rahmen angeordnet ist.

12. Scheibenförmiges Augment nach einem der vorangehenden Ansprüche, **dadurch gekennzeichnet, dass** die Durchgangsöffnung (2) ein langgestreckter Aufnahmeraum für einen Verankerungskiel (8) ist, und/oder die Durchgangsöffnung (2) zu einer Seite hin offen ist, und/oder die Durchgangsöffnung weniger als die Hälfte, vorzugsweise weniger als ein Drittel des Volumens des Augments einnimmt.

13. Scheibenförmiges Augment nach einem der vorangehenden Ansprüche, **dadurch gekennzeichnet, dass** der Außenmantel (15) konisch geneigt ist, vorzugsweise mit einem Winkel von 5 bis 10° nach distal verjüngend.

14. Scheibenförmiges Augment nach einem der vorangehenden Ansprüche, **dadurch gekennzeichnet, dass** an dem Außenmantel (15) radial nach außen gerichtete Spikes (16) vorgesehen sind.

15. Scheibenförmiges Augment nach einem der vorangehenden Ansprüche, **dadurch gekennzeichnet, dass** die geriffelte Struktur (40) genutet ausgeführt ist, mit vorzugsweise durchlaufenden Nuten (42).

16. Scheibenförmiges Augment nach einem der vorangehenden Ansprüche, **dadurch gekennzeichnet, dass** die geriffelte Struktur (40) durch ein Drahterodier-Verfahren hergestellt und insbesondere scharfkantig ausgeführt ist, und/oder das Augment einheitlich durch ein additives Verfahren, insbesondere 3D-Druck, hergestellt ist.

17. Scheibenförmiges Augment nach einem der vorangehenden Ansprüche, **dadurch gekennzeichnet, dass** die Deckplatte (4) zementdicht ausgeführt ist.

18. Scheibenförmiges Augment nach einem der vorangehenden Ansprüche, **dadurch gekennzeichnet, dass** die poröse Struktur (5) mit einer knocheneinwuchsfördernden Beschichtung (55), insbesondere umfassend Kalziumphosphat, versehen ist, und/oder an nicht-porösen Bereichen mit einer bioziden Beschichtung, insbesondere umfassend Silber, versehen ist.

19. Anordnung aus einer Endoprothese, insbesondere einer Kniegelenkendoprothese, und dem scheibenförmigen Augment (1) nach einem der vorangehenden Ansprüche, **dadurch gekennzeichnet, dass** ein Verankerungskiel (8) der Endoprothese (9) in die Durchgangsöffnung (2) aufgenommen ist.

20. Anordnung nach Anspruch 19, **dadurch gekennzeichnet, dass** das scheibenförmige Augment (1) außenkonturähnlich aber untermaßig bezogen auf die Endoprothese, insbesondere Tibiaplatte (91), ist.

21. Anordnung nach Anspruch 19 oder 20, **dadurch gekennzeichnet, dass** das scheibenförmige Augment (1) zur Unterstützung einer linken oder rechten Hälfte der Endoprothese (9) halbseitig ausgeführt ist.

22. Anordnung nach einem der Ansprüche 19 bis 21, **dadurch gekennzeichnet, dass** die Durchgangsöffnung (2) zur Aufnahme von dem Verankerungskiel (8) und dessen flügelartigen Auslegern (83) mit einem definierten Freiraum von vorzugsweise 1 bis 3 mm bemessen ist.

## Claims

1. A disk-shaped augment for filling bone defects, in particular at the end of tubular bones such as the tibia, with a proximal side (12), a distal side (11), an outer sheath (15) on lateral sides and an inner wall for a through-opening (2), runnning from the proximal side to the distal side, for an anchoring keel (8) of an endoprosthesis (9) arranged on the proximal side, wherein the augment has a generally C-shaped form with two legs (31, 32) flanking the through-opening,
**characterized in that**
a connecting piece (30) between the legs (31, 32) is formed in an articulated manner and interacts resiliently with the legs (31, 32) so that when the legs (31, 32) are compressed, an outwardly directed restoring force is generated, and
the outer casing (15) is designed as a porous structure (5) which promotes bone ingrowth, and
the proximal side (12) is provided with a cover plate (4) which has a ribbed structure (40) as an interlocking means for a cement connection.

2. The disk-shaped augment according to claim 1, **characterized in that** the distal side (11) is designed as a porous structure (5), wherein edges (50) are preferably designed to be solid.

3. The disk-shaped augment according to claim 1 or 2, **characterized in that** the porous structure (5) comprises pores connected to one another in the depth of the material, which preferably have a width of 0.4 to 1.0 mm.

4. The disk-shaped augment according to anyone of the preceding claims, **characterized in that** the porous structure (5) has porous areas which are framed by a solid edge.

5. The disk-shaped augment according to anyone of the preceding claims, **characterized in that** the legs (31, 32) have a solid core (33) on the outside of which pockets (35) are formed, in which the porous structure (5) is arranged.

6. The disk-shaped augment according to anyone of claims 1 to 4, **characterized in that** the legs (31, 32) are formed from a porous structure (5).

7. The disk-shaped augment according to anyone of the preceding claims, **characterized in that** the inner walls on the legs (31, 32) are solid.

8. The disk-shaped augment according to anyone of the preceding claims, **characterized in that** the legs (31, 32) are designed in a skeletal design with multiple disk segments (36) lying next to one another, separated by slots (34), and arranged on a frame (39) via flexible webs (37), wherein the frame (39) is preferably designed as an outer edge which encloses at least half of the legs (31, 32) and on the outside of which the outer sheath (15) is arranged.

9. The disc-shaped augment according to claim 7 or 8, **characterized in that** the slots (34) are dimensioned so narrowly that they act as a gap seal for bone cement, having preferably a maximum width of 1 mm, more preferably a maximum width of 0.7 mm.

10. The disk-shaped augment according to anyone of the preceding claims, **characterized in that** at least one fastening hole (28) for receiving a fastening screw (29) is provided in the legs (31, 32), wherein the fastening hole (27) preferably has a solid perforated casing (27).

11. The disk-shaped augment according to claim 10, **characterized in that** the fastening hole (28) is arranged in anyone of the disk segments (36) according to claim 7, 8 or 9, wherein this disk segment (36) is arranged on the frame by means of a bridge (38) which is at least twice as wide as the webs (37).

12. The disk-shaped augment according to anyone of the preceding claims, **characterized in that** the through-opening (2) is an elongate receiving space for an anchoring keel (8) and/or the through-opening (2) is open on one side and/or the through-opening occupies less than half, preferably less than a third, of the volume of the augment.

13. The disk-shaped augment according to anyone of the preceding claims, **characterized in that** the outer sheath (15) is conically inclined, preferably tapering distally at an angle of 5 to 10°.

14. The disk-shaped augment according to anyone of the preceding claims, **characterized in that** radially outwardly directed spikes (16) are provided on the outer casing (15) .

15. The disk-shaped augment according to anyone of the preceding claims, **characterized in that** the corrugated structure (40) is designed with grooves, preferably with continuous grooves (42).

16. The disk-shaped augment according to anyone of the preceding claims, **characterized in that** the ribbed structure (40) is produced by a wire-cut EDM process and is in particular designed with sharp edges, and/or the augment is produced uniformly by an additive process, in particular 3D printing.

17. The disk-shaped augment according to anyone of the preceding claims, **characterized in that** the cover plate (4) is designed to be cement-tight.

18. The disk-shaped augment according to anyone of the preceding claims, **characterized in that** the porous structure (5) is provided with a bone ingrowth-promoting coating (55), in particular comprising calcium phosphate, and/or on non-porous areas with a biocidal coating, in particular comprising silver.

19. An arrangement of an endoprosthesis, in particular of a knee joint endoprosthesis, and the disk-shaped augment (1) according to anyone of the preceding claims, **characterized in that** an anchoring keel (8) of the endoprosthesis (9) is accommodated in the through-opening (2).

20. The arrangement according to claim 19, **characterized in that** the disk-shaped augment (1) is similar in outer contour but undersized in relation to the endoprosthesis, in particular the tibial plate (91).

21. The arrangement according to claim 19 or 20, **characterized in that** the disc-shaped augment (1) is designed to support a left or right half of the endoprosthesis (9) on one half of the side.

22. The arrangement according to anyone of claims 19 to 21, **characterized in that** the through-opening (2) for receiving the anchoring keel (8) and its wing-like extensions (83) is dimensioned with a defined free space of preferably 1 to 3 mm.

## Revendications

1. Insert en forme de disque pour combler les défauts osseux, en particulier à l'extrémité des os longs tubulaires tels que le tibia, avec un côté proximal (12), un côté distal (11), une enveloppe extérieure (15) sur les côtés latéraux et une paroi intérieure pour une ouverture traversante évoluant du côté proximal au côté distal (2) pour une quille d'ancrage (8) d'une endoprothèse (9) agencée sur le côté proximal, dans lequel l'augmentation ayant une forme générale en C avec deux pattes (31, 32) encadrant l'ouverture traversante,
**caractérisé en ce**
**qu'**une pièce de liaison (30) entre les pattes (31, 32) est conçue de manière articulée et coopère élastiquement avec les pattes (31, 32), de sorte que, en cas de compression des pattes (31, 32), une force de rappel dirigée vers l'extérieur est générée, et
**que** l'enveloppe extérieure (15) est conçue comme une structure poreuse (5) qui favorise la croissance osseuse, et
**que** le côté proximal (12) est pourvu d'une plaque de couverture (4) qui présente une structure ondulée (40) en tant que dents pour une liaison de ciment.

2. Insert en forme de disque selon la revendication 1, **caractérisé en ce que** la face distale (11) est conçue comme une structure poreuse (5), dans lequel les bords (50) sont de préférence conçus de manière massive.

3. Insert en forme de disque selon la revendication 1 ou 2, **caractérisée en ce que** la structure poreuse (5) comprend des pores reliés les uns aux autres dans la profondeur du matériau, qui présentent de préférence une largeur de 0,4 à 1,0 mm.

4. Insert en forme de disque selon l'une des revendications précédentes, **caractérisé en ce que** la structure poreuse (5) présente des zones poreuses qui sont encadrées par un bord massif.

5. Insert en forme de disque selon l'une des revendications précédentes, **caractérisé en ce que** les pattes (31, 32) présentent un noyau massif (33) à l'extérieur duquel sont formées des poches (35), dans lesquelles la structure poreuse (5) est disposée.

6. Insert en forme de disque selon l'une des revendications 1 à 4, **caractérisé en ce que** les pattes (31, 32) sont formées à partir d'une structure poreuse (5) .

7. Insert en forme de disque selon l'une des revendications précédentes, **caractérisé en ce que** les parois intérieures sont conçues de manière massive au niveau des pattes (31, 32) .

8. Insert en forme de disque selon l'une des revendications précédentes, **caractérisé en ce que** les pattes (31, 32) sont conçues dans une conception de squelette avec une pluralité de segments de disque (36) se trouvant les uns à côté des autres, séparés par des fentes (34), lesquels segments sont disposés au niveau d'un châssis (39) par des traverses flexibles (37), dans lequel le châssis (39) est de préférence conçu comme un bord extérieur qui entoure au moins la moitié des pattes (31, 32) et à l'extérieur duquel l'enveloppe extérieure (15) est disposée.

9. Insert en forme de disque selon la revendication 7 ou 8, **caractérisé en ce que** les fentes (34) sont dimensionnées si étroitement qu'elles agissent comme un joint d'étanchéité pour le ciment osseux, de préférence d'un maximum de 1 mm de large, de manière davantage préférée d'un maximum de 0,7 mm.

10. Insert en forme de disque selon l'une des revendications précédentes, **caractérisé en ce qu'**au moins un trou de fixation (28) destiné à recevoir une vis de fixation (29) est prévu dans les pattes (31, 32), dans lequel le trou de fixation (27) présente de préférence un boîtier perforé solide (27).

11. Insert en forme de disque selon la revendication 10, **caractérisé en ce que** le trou de fixation (28) est disposé dans l'un des segments de disque (36) selon la revendication 7, 8 ou 9, dans lequel ce segment de disque (36) est disposé sur le châssis au moyen d'un pont (38) au moins deux fois plus large que les traverses (37).

12. Insert en forme de disque selon l'une des revendications précédentes, **caractérisé en ce que** l'ouverture traversante (2) est un espace allongé de réception pour une quille d'ancrage (8), et/ou que l'ouverture traversante (2) est ouverte sur un côté, et/ou que l'ouverture traversante occupe moins de la moitié, de préférence moins du tiers, du volume de l'insert.

13. Insert en forme de disque selon l'une des revendications précédentes, **caractérisé en ce que** l'enveloppe extérieure (15) est inclinée coniquement, de préférence s'effilant distalement à raison d'un angle de 5 à 10°.

14. Insert en forme de disque selon l'une des revendications précédentes, **caractérisé en ce que** des pointes (16) dirigées radialement vers l'extérieur sont prévues sur l'enveloppe extérieure (15).

15. Insert en forme de disque selon l'une des revendications précédentes, **caractérisé en ce que** la structure ondulée (40) est conçue avec des rainures, de préférence avec des rainures continues (42).

16. Insert en forme de disque selon l'une des revendications précédentes, **caractérisé en ce que** la structure ondulée (40) est réalisée par un procédé d'électroérosion à fil et est notamment conçue avec des arêtes vives, et/ou que l'augmentation est conçue uniformément par un procédé additif, en particulier l'impression 3D.

17. Insert en forme de disque selon l'une des revendications précédentes, **caractérisé en ce que** la plaque de couverture (4) est conçue pour être étanche au ciment.

18. Insert en forme de disque selon l'une des revendications précédentes, **caractérisé en ce que** la structure poreuse (5) est munie d'un revêtement favorisant la croissance osseuse (55), comprenant en particulier du phosphate de calcium, et/ou est munie, sur les zones non poreuses, d'un revêtement biocide, comprenant en particulier de l'argent.

19. Disposition d'une endoprothèse, en particulier d'une endoprothèse d'articulation de genou, et de l'insert en forme de disque (1) selon l'une des revendications précédentes, **caractérisée en ce qu'**une quille d'ancrage (8) de l'endoprothèse (9) est reçue dans l'ouverture traversante (2).

20. Disposition selon la revendication 19, **caractérisée en ce que** l'insert en forme de disque (1) est de contour extérieur similaire mais sous-dimensionné par rapport à l'endoprothèse, en particulier le plateau tibial (91).

21. Disposition selon la revendication 19 ou 20, **caractérisée en ce que** l'insert en forme de disque (1) est conçu pour supporter une moitié gauche ou droite de l'endoprothèse (9) d'un côté.

22. Disposition selon l'une des revendications 19 à 21, **caractérisée en ce que** l'ouverture traversante (2) pour la réception de la quille d'ancrage (8) et de ses prolongements en forme d'ailes (83) est dimensionnée à raison d'un espace libre défini de préférence de 1 à 3 mm.
